(19) Europäisches Patentamt
European Patent Office
Office européen des brevets

(11) Veröffentlichungsnummer: **0 163 596**
**B1**

(12) # EUROPÄISCHE PATENTSCHRIFT

(45) Veröffentlichungstag der Patentschrift: **16.08.90**

(51) Int. Cl.[5]: **C 07 J 1/00,** A 61 K 31/565, C 07 J 51/00

(21) Anmeldenummer: **85730070.1**

(22) Anmeldetag: **10.05.85**

(54) **Estriolester.**

(30) Priorität: **16.05.84 DE 3418562**

(43) Veröffentlichungstag der Anmeldung:
**04.12.85 Patentblatt 85/49**

(45) Bekanntmachung des Hinweises auf die Patenterteilung:
**16.08.90 Patentblatt 90/33**

(84) Benannte Vertragsstaaten:
**AT BE CH DE FR GB IT LI LU NL SE**

(56) Entgegenhaltungen:
**GB-A- 879 014**
**GB-A-1 006 802**

**CHEMICAL ABSTRACTS, Band 72, Nr. 23, 8. Juni 1970, Seite 384, Nr. 121768b, Columbus, Ohio, US; T. NAMBARA u.a.: "Analytical chemical studies on steroids. XXXIV. Acyl transfer during borohydride reduction of steroidal 16,17-ketol acetates" & CHEM. PHARM. BULL. 1970, 18(3), 626-9**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Laurent, Henry, Dr.**
**Glambecker Weg 21**
**D-1000 Berlin 28 (DE)**
Erfinder: **Bittler, Dieter**
**Bölkauer Pfad 11**
**D-1000 Berlin 27 (DE)**
Erfinder: **Beier, Sybille, Dr.**
**Uhlandstrasse 121**
**D-1000 Berlin 31 (DE)**
Erfinder: **Elger, Walter, Dr.**
**Schorlemer Allee 12 B**
**D-1000 Berlin 33 (DE)**

**EP 0 163 596 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die Erfindung betrifft neue Estriolester der allgemeinen Formel I

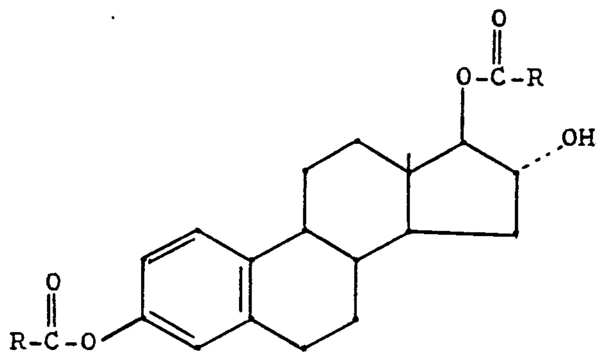

(I),

worin R jeweils den Rest einer Monocarbonsäure mit 2 bis 10 Kohlenstoffatomen bedeutet, sowie Verfahren zu ihrer Herstellung und pharmazeutische Präparate, die die neuen Estriolester enthalten.

Estriol mit der chemischen Bezeichnung 1,3,5(10)-Estratrien-3,16α,17β-triol ist ein wichtiges natürliches Östrogen.

Estriol wird auch als wirksamer Bestandteil in Präparaten zur Substitution von Östrogen bei Östrogenmangelerscheinungen angewandt, beispielsweise bei Frauen in der Postmenopause.

Da Estriol sehr rasch aus dem Körper ausgeschieden wird, muß es in kurzen Abständen (1 bis 3 mal täglich) appliziert werden.

In der Literatur sind auch einige Ester von Estriol beschrieben, beispielsweise Estriol-triester, 16,17-Diester und 16-Monoester in Chem. Pharm. Bull. 11 (1963) 510—514, ferner 3-Acetat, 3,16-Diacetat und 16,17-Diacetat in Acta Chem. Scand. 22 (1968) 254.

Aus der GB—A—1,006,802 ist das 3,16(α),17(β)-Tripropionat des Estriols bekannt. Verglichen mit dem Estriol besitzt dieser Ester eine höhere östrogene Wirksamkeit und eine wesentlich höhere Wirkungsdauer. Die maximale Wirkung des aus dem Ester gebildeten Estriols tritt jedoch relativ frühzeitig nach der Anwendung auf, danach fällt die Wirkung wieder beträchtlich ab, etwa mit einem Drittel der Geschwindigkeit des Anstiegs.

Mit Ausnahme von Estriolsuccinat, das täglich 3 mal appliziert werden muß, sind keine Estriolester medizinisch angewendet worden.

Die bisher nicht beschriebenen 3,17β-Diester des Estriols mit Monocarbonsäuren mit 2 bis 10 Kohlenstoffatomen übertreffen Estriol in der Stärke und Dauer der Östrogenwirkung.

Die Esterreste R in der allgemeinen Formel I sind vorwiegend gleich und leiten sich von einer aliphatischen, cycloaliphatisch-aliphatischen oder aromatischen Monocarbonsäure ab. Bevorzugte Esterreste R sind die von Essigsäure, Propionsäure, Buttersäure, Isobuttersäure, Pivalinsäure, Valeriansäure, Capronsäure, Önanthsäure, Octan- und Decansäure, ferner β-Cyclopentylpropionsäure und Benzoesäure.

Die östrogene Aktivität und die Depoteigenschaften der neuen Estriolester wurden im Vergleich zu Estriol ($E_3$) im modifizierten Uteruswachstumstest nach Rubin an ovariektomierten Ratten bestimmt (Endocrinology *49* (1951) 429—439).

Adulte ovariektomierte Ratten im Gewicht von ca. 150 g, 6 Tiere pro Dosisgruppe, werden einmal mit der jeweiligen Test- bzw. Referenzsubstanz (Estriol) behandelt. Der Tag der Substanzgabe gilt als Tag 1 ($d_1$) des Versuchs, Die Substanzen werden in einem Gemisch aus Benzylbenzoat + Rizinusöl im Verhältnis 4:6 gelöst und die Tagesdosis in einem Volumen von 0,2 ml subcutan (s.c.) appliziert. Einer Kontrollgruppe werden nur 0,2 ml Vehikel verabbreicht.

Wirksame Östrogene führen bei ovariektomierten Ratten zu charakteristischen Veränderungen am Vaginalepithel. Es kommt zu einer starken Proliferation des Vaginalepithels und zur Verhornung des oberflächlichen Zellagen. Es werden täglich einmal Vaginalabstriche vorgenommen. Die Abstrichbilder werden cytologisch beurteilt.

Folgende Zyklusstadien werden unterschieden:

1 = Diöstrus (Leukozyten und kernhaltige Epithelzellen),
2 = Proöstrus (kernhaltige Epithelzellen),
3 = Östrus (kernlose Hornschollen),
4 = Metöstrus (kernlose Hornschollen, Leukozyten, Epithelzellen).

Zur Ermittlung der Dauer der Östrogenwirkung an der Vagina wird die Zeit in Tagen bestimmt, an denen der Östrus aufrechterhalten wird.

Aus Tabelle 1 ist zu entnehmen, daß nach Applikation von Estriol ($E_3$) die Tiere einen Tag im Östrus bleiben; nach Applikation equimolarer Mengen von $E_3$-Dipropionat, $E_3$-Dibutyrat, $E_3$-Diisobutyrat, $E_3$-Divalerianat und $E_3$-Dihexanoat hält der Östrus 8, 16, 23, 28 bzw. 27 Tage an.

TABELLE 1

Dauer der Östrogenwirkung an der Vagina nach einmaliger Injektion (s.c.) von Estriol ($E_3$) bzw. jeweils einmalioger Injektion von verschiedenen 3,17-Diestern bei ovariektomierten Ratten in equimolaren Dosen

| Verbindung | Östrus |
|---|---|
| Estriol ($E_3$) 100 µg s.c. | 1 Tag |
| $E_3$-Dipropionat 139 µg s.c. | 8 Tage |
| $E_3$-Dibutyrat 149 µg s.c. | 16 Tage |
| $E_3$-Diisobutyrat 149 µg s.c. | 23 Tage |
| $E_3$-Divalerianat 158 µg s.c. | 28 Tage |
| $E_3$-Dihexanoat 168 µg s.c. | 27 Tage |

In Tabelle 2 wird der zeitliche Verlauf der Estriol ($E_3$)-Serumkonzentration in pmol/l nach einmaliger Injektion (s.c.) von Estriol ($E_3$) und jeweils einmaliger Injektion (s.c.) von verschiedenen $E_3$-3,17-Diestern bei ovariektomierten Ratten angegeben.

Am 1. Tag, 2 Stunden vor der Injektion, am 5., 10., 15., 20., 25. und 30. Tag nach der Injektion wird den Tieren Blut abgenommen, um die Serum-$E_3$-Konzentration mittels RIA (Radio Immuno Assay) zu bestimmen.

Aus Tabelle 2 geht hervor, daß nach Applikation von Estriol-3,17-Diestern die radioimmunologisch gemessene $E_3$-Konzentration 5 bis 15 mal höher liegt als nach equimolarer Applikation von Estriol ($E_3$).

Bei den Diestern werden erhöhte $E_3$-Konzentrationen bis zu 30 Tagen nach der Applikation beobachtet, bei Estriol nur 8 Tage lang.

TABELLE 2

Zeitlicher Verlauf der $E_3$-Serumkonzentration [pmol/l] nach einmaliger Injektion (s.c.) von Estriol ($E_3$) bzw. jeweils einmaliger Injektion (s.c.) von verschiedenen 3,17-Diestern bei ovariektomierten Ratten in equimolaren Dosen

| Verbindung | Tag 1*) | Tag 5 | Tag 10 | Tag 15 | Tag 20 | Tag 25 | Tag 30 |
|---|---|---|---|---|---|---|---|
| Estriol ($E_3$) 100 µm s.c. | 37,2 | 63,6 | Tag 8: 57,0 | | | | |
| $E_3$-Dipropionat 139 µg s.c. | 49,2 | 1047,9 | 159,2 | 106,8 | | | |
| $E_3$-Dibutyrat 149 µg s.c. | 41,0 | 990,5 | 251,5 | 124,0 | 62,5 | Tag 24: 46,7 | |
| $E_3$-Diisobutyrat 149 µg s.c. | 34,0 | 691,7 | 280,7 | 213,2 | 96,5 | 77,8 | 50,7 |
| $E_3$-Divalerianat 158 µg s.c. | 39,7 | 600,8 | 264,2 | 209,3 | 150,2 | 85,7 | 75,8 |
| $E_3$-Dihexanoat 168 µg s.c. | 46,5 | 335,9 | 367,8 | 259,8 | 206,5 | 199,1 | 89,2 |
| $E_3$-Didecanoat 207 µg s.c. | 55,7 | 179,0 | 188,8 | 170,6 | 155,0 | 109,0 | 92,9 |
| Lösungsmittelkontrolle s.c. | 39,9 | 39,8 | 39,3 | 37,1 | 36,7 | 36,0 | 50,3 |

*) 2 Stunden vor der Injektion

Aus den tierexperimentellen Ergebnissen ist ersichtlich, daß durch Veresterung von Estriol sowohl die Wirkungsstärke als auch die Wirkungsdauer ansteigen.

Durch Veresterung von Estriol in 3,17-Stellung wird eine stoffwechsel-stabilisierte Form eines natürlichen Östrogens erhalten, das medizinisch vielfältig anwendbar ist. Obwohl die neuen Ester auch oral verabreicht werden können, ist die parenterale Applikationsmethode bevorzugt, weil erst dann die Vorteile der neuen Ester voll in Erscheinung treten. Durch parenterale Applikation wird die erste Leberpassage und dadurch die rasche Metabolisierung vermieden. Weiterhin werden durch parenterale Applikation die schädlichen hepatischen Östrogenwirkungen vermieden, wie zum Beispiel der Anstieg der Gerinnungsfaktoren, der Anstieg der Hormontransportproteine, die Zunahme der Angiotensinogene und die Gleichgewichtsverschiebung der Lipoproteine.

Hauptanwendungsgebiete der neuen Estriolester sind die Substitution von Östrogen bei Frauen in der Postmenopause, die unter klimakterischen Ausfallserscheinungen wie Hitzewallungen, Osteoporose, Haut- und Genitalatrophie leiden, ferner die Fertilitätskontrolle bei der Frau und die gynäkologischen Indikationen, wie zum Beispiel Vaginalatrophie, Kraurosis vulvae usw.

Die zu verabreichende Menge der neuen Estriolester schwankt innerhalb eines weiten Bereichs und kann jede wirksame Menge abdecken. In Abhängigkeit des zu behandelnden Zustands, der Art und Häufigkeit der Verabreichung kann die Menge etwa 10 bis 300 mg betragen.

Die neuen Ester sind als Prodrug von Estriol zur Herstellung injizierbarer oder implantierbarer Depotpräparate besonders geeignet. Sie haben gegenüber den oral applizierbaren Präparaten auch den Vorteil, daß eine einzige Injektion für einen oder mehrere Monate ausreichend ist, während zum Beispiel Tabletten täglich eingenommen werden müssen. Die Dauer der Depotwirkung hängt von der Kettenlänge und der Menge des Esters ab sowie von der Art der Trägersubstanz, die den Wirkstoff freigibt.

Als Trägersubstanzen sind physiologisch verträgliche Verdünnungsmittel geeignet, in denen die Wirkstoffe gelöst oder suspendiert sein können. Als Verdünnungsmittel ist zum Beispiel Wasser mit oder ohne Zusatz von Elektrolytsalzen oder Verdickungsmitteln geeignet. So kann die Depotformulierung zum Beispiel eine wäßrige Mikrokristallsuspension sein.

Sehr häufig werden als Verdünnungsmittel auch Öle mit oder ohne Zusatz eines Lösungsvermittlers, eines oberflächenaktiven Stoffes, eines Suspendier- oder Emulgiermittels verwendet. Beispiele für verwendete Öle sind insbesondere Olivenöl, Erdnußöl, Baumwollsamenöl, Sojabohnenöl, Rizinusöl und Sesamöl. Beispiele für Lösungsvermittler sind insbesondere Benzylalkohol und Benzylbenzoat. Eine bevorzugte Mischung besteht aus 6 Gewichtsteilen Rizinusöl und 4 Gewichtsteilen Benzylbenzoat.

Bei der Verwendung der neuen Estriolester als Depotkontrazeptiva können die neuen Ester mit einem Depotgestagen oder einem oral zu verabreichenden Gestagen kombiniert werden. Die kombinierte Anwendung kann gleichzeitig oder zeeitlich versetzt erfolgen. So kann man beispielsweise ein Depotöstrogen der allgemeinen Formel I und ein Depotgestagen zu einer 1-Monatsspritze kombinieren. Als Depotgestagen für eine ölige Lösung ist zum Beispiel Norethisteronönanthat und für eine Mikrokristallsuspension Medroxyprogesteronacetat geeignet.

Je nach der gewünschten Dauer der Wirkung können etwa 10 bis 300 mg des neuen Depotöstrogens mit 30 bis 300 mg eines Depotgestagens kombiniert werden.

Man kann auch das erfindungsgemäße Depotöstrogen injizieren und ein übliches Gestagen wie Norethisteron, Norgestrel, Levonorgestrel oder Cyproteronacetat täglich oral applizieren.

Implantationspräparate können den Wirkstoff mit inerten Materialien, zum Beispiel biologisch abbaubaren Polymeren, enthalten. Die Wirkstoffe können auch mit Silikonkautschuck zu Implantaten verarbeitet werden.

Die neuen Verbindungen der allgemeinen Formel I werden hergestellt, indem man einen Estriol-16-silylether der allgemeinen Formel II

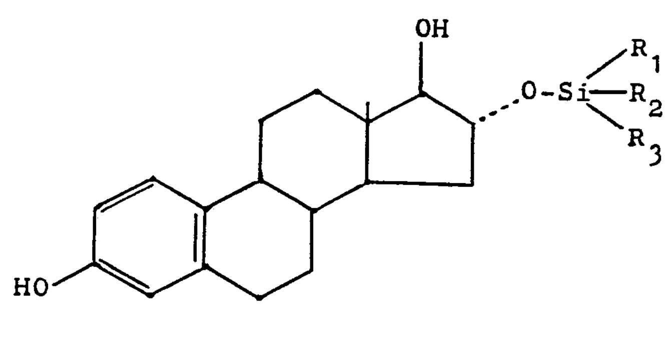

(II),

worin $R_1$, $R_2$, $R_3$ gleich oder verschieden sind und jeweils Alkyl mit 1 bis 5 Kohlenstoffatomen, Phenyl oder Benzyl bedeuten, in an sich bekannter Weise in 3- und 17-Stellung verestert und anschließend den Silylether spaltet.

Die Veresterung der Hydroxygruppen in 3- und 17β-Stellung des Estriol-16-silylethers erfolgt in an sich bekannter Weise mit der entsprechenden Monocarbonsäure RCOOH oder einem Derivat, insbesondere dem Anhydrid oder Chlorid der Monocarbonsäure in Gegenwart einer Base. Als Basen kommen

insbesondere tertiäre Amine, wie Pyridin, 4-Dimethylaminopyridin, Collidin, Triethylamin oder Gemische aus diesen Aminen, infrage.

Die sich anschließende Spaltung des Silylethers wird ebenfalls nach bekannten Methoden durchgeführt. Eine bevorzugte Methode ist die Spaltung mit Tetrabutylammoniumfluorid in wasserfreiem Tetrahydrofuran bei Raumtemperatur.

Die als Ausgangsverbindungen benutzten Estriol-16-silylether der allgemeinen Formel II werden durch Umsetzung von Estriol mit dem entsprechenden Silylchlorid in Gegenwart von Imidazol in Dimethylformamid hergestellt. Als Silylchloride kommen beispielsweise tert.-Butyldimethylsilylchlorid, Dimethyl-2-(3-methylbutyl)-silylchlorid, Trimethyl-, Triphenyl- und Tribenzylsilylchlorid infrage.

Herstellung der Ausgangsverbindung

16α-(tert.-Butyl-dimethylsilyloxy)-1,3,5(10)-estratrien-3,17β-diol

Eine auf −20°C gekühlte Lösung von 11,52 g 1,3,5(10)-Estratrien-3,16α,17β-triol in 200 ml Dimethylformamid wird nach Zugabe von 6,52 g Imidazol tropfenweise mit einer Lösung von 13,24 g tert.-Butyl-dimethylsilylchlorid in 100 m Dimethylformamid versetzt. Dieses Reaktionsgemisch wird 1 Stunde unter weiterer Kühung gerührt und anschließend in Eiswasser gegossen. Der entstandene Niederschlag wird abfiltriert, mit Wasser gewaschen und in Dichlormethan gelöst. Die Lösung wird über Natriumsulfat getrocknet und im Vakuum eingedampft. Der Rückstand wird an Kieselgel chromatographiert.

Ausbeute: 11,30 g 16α-(tert.-Butyl-dimethylsilyloxy)-1,3,5(10)-estratrien-3,17β-diol.

Schmelzpunkt: 194°C.

Beispiel 1

a) Eine Lösung von 750 mg 16α-(tert.-Butyl-dimethylsilyloxy)-1,3,5(10)-estratrien-3,17β-diol in 3 ml Pyridin wird mit 1,5 ml Acetanhydrid versetzt. Nach einer Reaktionszeit von 20 Stunden bei 20°C wird die Lösung in Eiswasser gegossen. Der ausgefällte Niederschlag wird abfiltriert, mit Wasser gewaschen und getrocknet. Man erhält 950 mg 3,17β-Diacetoxy-16α-(tert.-butyl-dimethylsilyloxy)-1,3,5(10)-estratrien.

b) 950 mg 3,17β-Diacetoxy-16α-(tert.-butyl-dimethylsilyloxy)-1,3,5(10)-estratrien werden in 9,5 ml wasserfreiem Tetrahydrofuran gelöst. Die Lösung wird mit 950 mg Tetrabutylammoniumfluorid versetzt und 2,5-Stunden bei 20°C gerührt. Das Reaktionsgemisch wird mit Diethylether versetzt, mit Wasser gewaschen, über Natriumsulfat getrocknet und im Vakuum eingedampft. Der Rückstand wird an Kieselgel mit einem Pentan-Diethylether-Gradienten (0—20% Diethylether) chromatographiert.

Ausbeute: 310 mg 3,17β-Diacetoxy-1,3,5(10)-estratrien-16α-ol vom Schmelzpunkt 132°C (aus Diisopropylether).

$[\alpha]_D = +96°$ (in Chloroform).

Beispiel 2

a) Eine Lösung von 500 mg 16α-(tert.-Butyl-dimethylsilyloxy)-1,3,5(10)-estratrien-3,17β-diol in 2 ml Pyridin wird mit 1,0 ml Propionsäureanhydrid versetzt und 48 Stunden bei Raumtemperatur stehengelassen. Die Reaktionslösung wird mit Diethylether verdünnt, mit Wasser gewaschen, über Natriumsulfat getrocknet und im Vakuum eingedampft. Der Rückstand wird mit einem Pentan-Diethylether-Gradienten (0—20% Diethylether) chromatographiert.

Ausbeute: 660 mg 16α-(tert.-Butyl-dimethylsilyloxy)-3,17β-dipropionyloxy-1,3,5(10)-estratrien.

b) 660 mg 16α-(tert.-Butyl-dimethylsilyloxy)-3,17β-dipropionyloxy-1,3,5(10)-estratrien werden, wie in Beispiel 1b) beschrieben, mit Tetrabutylammoniumfluorid umgesetzt. Nach entsprechender Aufarbeitung erhält man 220 mg 3,17β-Dipropionyloxy-1,3,5(10)-estratrien-16α-ol vom Schmelzpunkt 105°C (aus Diisopropylether).

$[\alpha]_D^{22} = +90°$ (in Chloroform).

Beispiel 3

a) Die Lösung von 1,0 g 16α-(tert.-Butyl-dimethylsilyloxy)-1,3,5(10)-estratrien-3,17β-diol in 4 ml Pyridin und 2 ml Buttersäureanhydrid wird unter Zusatz von 100 mg 4-Dimethylaminopyridin 20 Stunden bei Raumtemperatur stehengelassen. Die Aufarbeitung erfolgt wie in Beispiel 2a) beschrieben. Man erhält 1,4 g 16α-(tert.-Butyl-dimethylsilyloxy)-3,17β-dibutyryloxy-1,3,5(10)-estratrien.

b) 1,4 g 16α-(tert.-Butyl-dimethylsilyloxy)-3,17β-dibutyryloxy-1,3,5(10)-estratrien werden, wie in Beispiel 1b) beschrieben, umgesetzt. Nach entsprechender Aufarbeitung erhält man 900 mg 3,17β-Dibutyryloxy-1,3,5(10)-estratrien-16α-ol als Öl.

$[\alpha]_D^{22} = +84°$ (in Chloroform).

Beispiel 4

a) 1,0 g 16α-(tert.-Butyl-dimethylsilyloxy)-1,3,5(10)-estratrien-3,17β-diol werden unter den in Beispiel 3a) beschriebenen Reaktionsbedingungen mit Isobuttersäureanhydrid umgesetzt. Man erhält 1,3 g 16α-(tert.-Butyl-dimethylsilyloxy)-3,17β-diisobutyryloxy-1,3,5(10)-estratrien.

b) Aus 1,3 g 16α-(tert.-Butyl-dimethylsilyloxy)-3,17β-diisobutyryloxy-1,3,5(10)-estratrien erhält man, unter den in Beispiel 1b) beschriebenen Bedingungen, 830 mg 3,17β-Diisobutyryloxy-1,3,5(10)-estratrien-16α-ol vom Schmelzpunkt 114°C.

$[\alpha]_D^{22} = +86°$ (in Chloroform).

Beispiel 5

a) Aus 1,0 g 16α-(tert.-Butyl-dimethylsilyloxy)-1,3,5(10)-estratrien-3,17β-diol erhält man mit Valeriansäureanhydrid, unter den in Beispiel 3a) beschriebenen Bedingungen, 1,37 g 16α-(tert.-Butyl-dimethylsilyloxy)-3,17β-divaleryloxy-1,3,5(10)-estratrien.

b) Unter den in Beispiel 1b) beschriebenen Bedingungen werden aus 1,37 g 16α-(tert.-Butyl-dimethylsilyloxy)-3,17β-divaleryloxy-1,3,5(10)-estratrien 760 mg 3,17β-Divaleryloxy-1,3,5(10)-estratrien-16α-ol als Öl erhalten.

$[\alpha]_D^{22} = +79°$ (in Chloroform).

Beispiel 6

a) Aus 750 mg 16α-(tert.-Butyl-dimethylsilyloxy)-1,3,5(10)-estratrien-3,17β-diol erhält man mit Capronsäureanhydrid, unter den in Beispiel 3a) beschriebenen Reaktionsbedingungen, 1,08 g 16α-(tert.-Butyl-dimethylsilyloxy)-3,17β-dihexanoyloxy-1,3,5(10)-estratrien.

b) Unter den in Beispiel 1b) beschriebenen Bedingungen werden aus 1,08 g 16α-(tert.-Butyl-dimethylsilyloxy)-3,17β-dihexanoyloxy-1,3,5(10)-estratrien 580 mg 3,17β-Dihexanoyloxy-1,3,5(10)-estratrien-16α-ol als Öl erhalten.

$[\alpha]_D^{22} = +75°$ (in Chloroform).

Beispiel 7

a) Aus 1,0 g 16α-(tert.-Butyl-dimethylsilyloxy)-1,3,5(10)-estrien-3,17β-diol erhält man mit Pivaloylchlorid, unter den in Beispiel 3a) beschriebenen Bedingungen, 1,38 g 16α-(tert.-Butyl-dimethylsilyloxy)-3,17β-dipivaloyloxy-1,3,5(10)-estratrien.

b) 1,38 g 16α-(tert.-Butyl-dimethylsilyloxy)-3,17β-dipivaloyloxy-1,3,5(10)-estratrien werden, unter den in Beispiel 1b) angegebenen Reaktionsbedingungen, in 1,10 g 3,17β-Dipivaloyloxy-1,3,5(10)-estratrien-16α-ol überführt.

Schmelzpunkt: 164°C.

$[\alpha]_D^{22} = +83°$ (in Chloroform).

Beispiel 8

a) Aus 750 mg 16α-(tert.-Butyl-dimethylsilyloxy)-1,3,5(10)-estratrien-3,17β-diol werden mit Decanoylchlorid, unter den in Beispiel 3a) beschriebenen Bedingungen, 1,10 g 16α-(tert.-Butyl-dimethylsilyloxy)-3,17β-didecanoyloxy-1,3,5(10)-estratrien erhalten.

b) 1,10 g 16α-(tert.-Butyl-dimethylsilyloxy)-3,17β-didecanoyloxy-1,3,5(10)-estratrien werden, unter den in Beispiel 1b) angegebenen Bedingungen, in 580 mg 3,17β-Didecanoyloxy-1,3,5(10)-estratrien-16α-ol überführt.

Schmelzpunkt: 64°C.

$[\alpha]_D^{22} = +63°$ (in Chloroform).

Beispiel 9

a) Aus 1,0 g 16α-(tert.-Butyl-dimethylsilyloxy)-1,3,5(10)-estratrien-3,17β-diol werden mit Benzoylchlorid, unter den in Beispiel 3a) beschriebenen Bedingungen, 1,34 g 3,17β-Dibenzoyloxy-16α-(tert.-butyl-dimethylsilyloxy)-1,3,5(10)-estratrien erhalten.

b) 1,34 g 3,17β-Dibenzoyloxy-16α-(tert.-butyl-dimethylsilyloxy)-1,3,5(10)-estratrien werden, unter den in Beispiel 1b) angegebenen Bedingungen, in 765 mg 3,17β-Dibenzoyloxy-1,3,5(10)-estratrien-16α-ol überführt.

**Patentansprüche für die Vertragsstaaten: BE CH LI DE FR GB IT LU NL SE**

1. Estriolester der allgemeinen Formel I

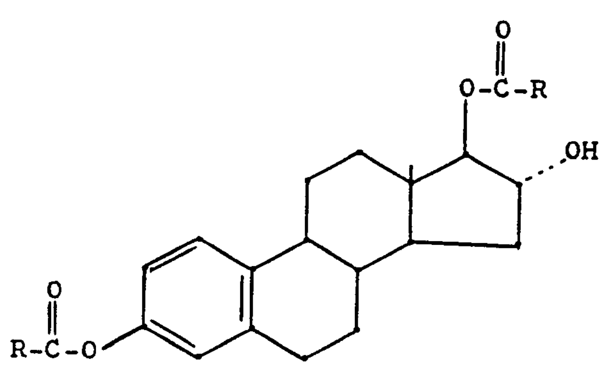

(I),

worin R jeweils den Rest einer Monocarbonsäure mit 2 bis 10 Kohlenstoffatomen bedeutet.

2. 3,17β-Diacetoxy-1,3,5(10)-estratrien-16α-ol.
3. 3,17β-Dipropionyloxy-1,3,5(10)-estratrien-16α-ol.
4. 3,17β-Diisobutyryloxy-1,3,5(10)-estratrien-16α-ol.
5. 3,17β-Divaleryloxy-1,3,5(10)-estratrien-16α-ol.
6. 3,17β-Dihexanoyloxy-1,3,5(10)-estratrien-16α-ol.
7. 3,17β-Dipivaloyloxy-1,3,5(10)-estratrien-16α-ol.
8. 3,17β-Didecanoyloxyoxy-1,3,5(10)-estratrien-16α-ol.
9. 3,17β-Dibenzoyloxy-1,3,5(10)-estratrien-16α-ol.
10. 3,17β-Dibutyryloxy-1,3,5(10)-estratrien-16α-ol.
11. Pharmazeutische Präparate, gekennzeichnet durch den Gehalt an einer Verbindung gemäß Anspruch 1 bis 10.
12. Depotkontrazeptiva, gekennzeichnet durch den Gehalt an einer Verbindung gemäß Anspruch 1 bis 10.
13. Verfahren zur Herstellung neuer Estriolester der allgemeinen Formel I

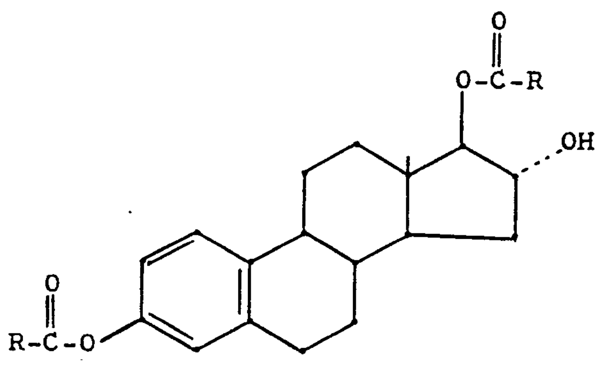

(I),

worin R jeweils den Rest einer Monocarbonsäure mit 2 bis 10 Kohlenstoffatomen bedeutet, dadurch gekennzeichnet, daß man einen Estriol-16-silylether der allgemeinen Formel II

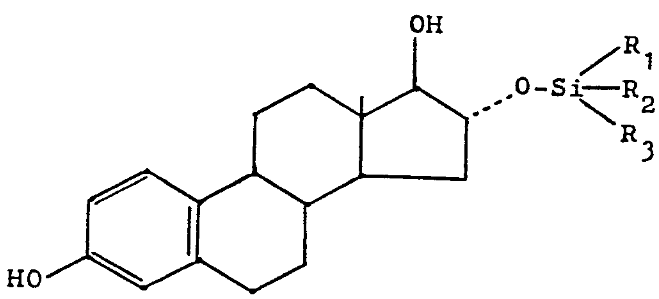

(II),

worin $R_1$, $R_2$, $R_3$ gleich oder verschieden sind und jeweils Alkyl mit 1 bis 5 Kohlenstoffatomen, Phenyl oder Benzyl bedeuten, in an sich bekannter Weise in 3- und 17-Stellung verestert und anschließend den Silylether spaltet.

**Patentanspruch für den Vertragsstaat: AT**

Verfahren zur Herstellung neuer Estriolester der allgemeinen Formel I

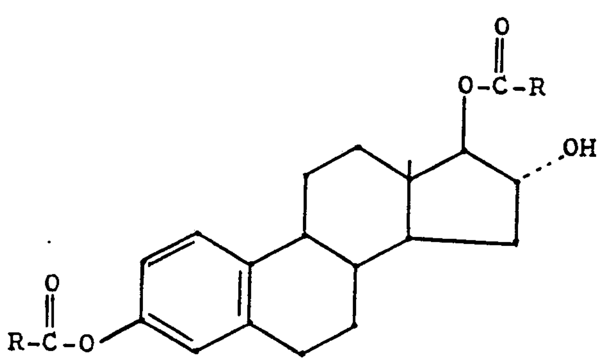

(I),

worin R jeweils den Rest einer Monocarbonsäure mit 2 bis 10 Kohlenstoffatomen bedeutet, dadurch gekennzeichnet, daß man einen Estriol-16-silylether der allgemeinen Formel II

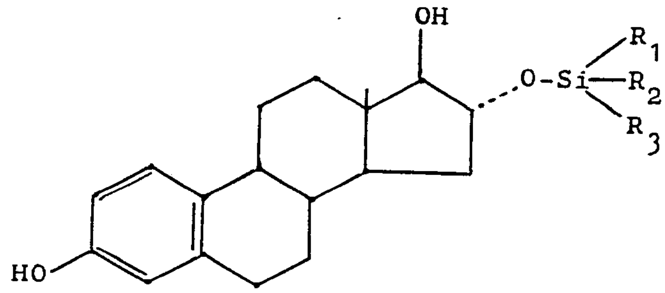

(II),

worin $R_1$, $R_2$, $R_3$ gleich oder verschieden sind und jeweils Alkyl mit 1 bis 5 Kohlenstoffatomen, Phenyl oder Benzyl bedeuten, in an sich bekannter Weise in 3- und 17-Stellung verestert und anschließend den Silylether spaltet.

**Revendications pour les Etats contractants: BE CH DE FR GB IT LI LU NL SE**

1. Esters d'oestriol répondant à la formule générale I

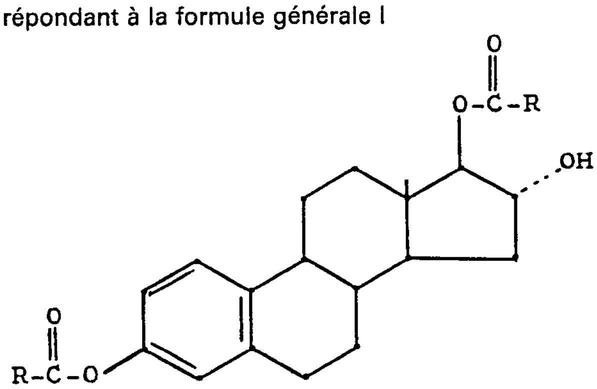

(I),

dans laquelle R représente chaque fois le radical d'un acide monocarboxylique comportant 2 à 10 atomes de carbone.

2. Le 3,17β-diacétoxy-1,3,5(10)-oestratrién-16α-ol.
3. Le 3,17β-dipropionyloxy-1,3,5(10)-oestratrién-16α-ol.
4. Le 3,17β-diisobutyryloxy-1,3,5(10)-oestratrién-16α-ol.
5. Le 3,17β-divaléryloxy-1,3,5(10)-oestratrién-16α-ol.
6. Le 3,17β-dihexanoyloxy-1,3,5(10)-oestratrién-16α-ol.
7. Le 3,17β-dipivaloyloxy-1,3,5(10)-oestratrién-16α-ol.
8. Le 3,17β-didécanoyloxyoxy-1,3,5(10)-oestratrién-16α-ol.
9. Le 3,17β-dibenzoyloxy-1,3,5(10)-oestratrién-16α-ol.
10. Le 3,17β-dibutyryloxy-1,3,5(10)-oestratrién-16α-ol.
11. Compositions pharmaceutiques, caractérisées par une teneur en un composé suivant l'une des revendications 1 à 10.
12. Compositions contraceptives retard, caractérisées par une teneur en un composé suivant l'une des revendications 1 à 10.
13. Procédé de préparation de nouveaux esters d'oestriol répondant à la formule générale I

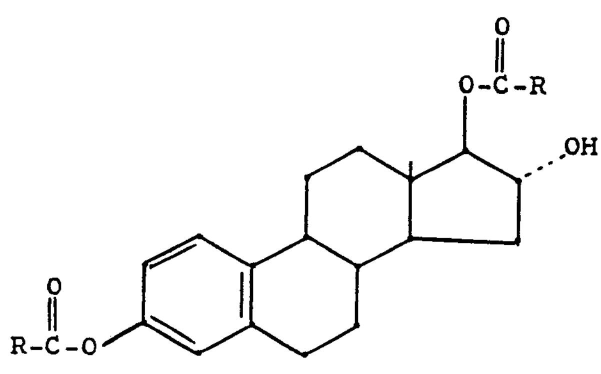

(I),

dans laquelle R représente chaque fois le radical d'un acide monocarboxylique comportant 2 à 10 atomes de carbone, caractérisé en ce qu'on estérifie d'une manière connue en soi dans les positions 3 et 17 un éther oestriol-16-silylique répondant à la formule générale II

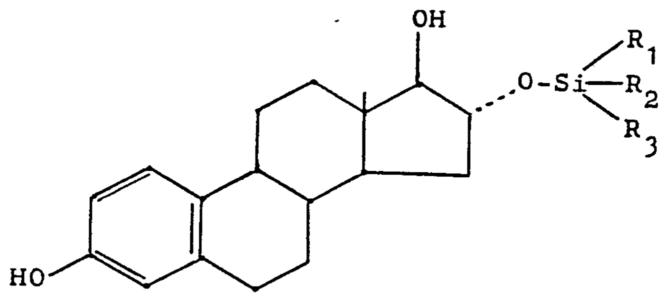

(II),

dans laquelle $R_1$, $R_2$, $R_3$ sont identiques ou différents et représentent chacun un radical alkyle comportant 1 à 5 atomes de carbone, phényle ou benzyle, et en ce qu'ensuite on sépare l'éther silylique.

**Revendication pour l'Etat contractant: AT**

Procédé de préparation de nouveaux esters d'oestriol répondant à la formule générale I

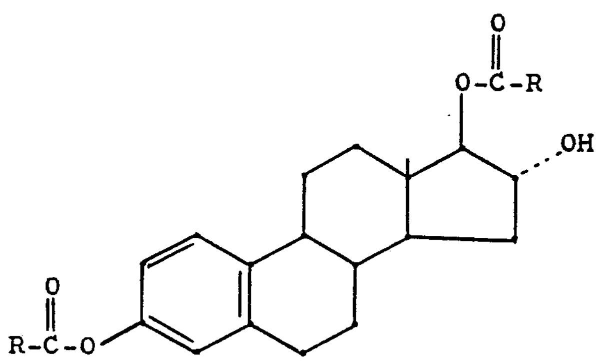

(I),

dans laquelle R représente chaque fois le radical d'un acide monocarboxylique comportant 2 à 10 atomes de carbone, caractérisé en ce qu'on estérifie d'une manière connue en soi dans les positions 3 et 17 un éther oestriol-16-silylique répondant à la formule générale II

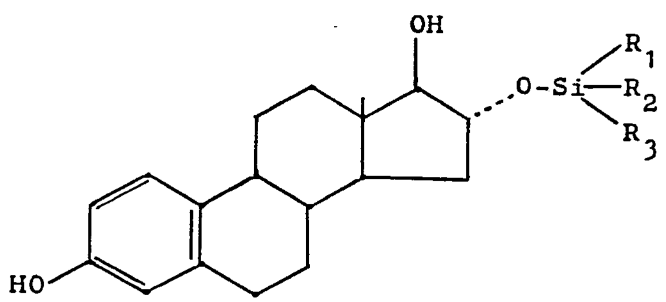

(II),

dans laquelle $R_1$, $R_2$, $R_3$ sont identiques ou différents et représentent chacun un radical alkyle comportant 1 à 5 atomes de carbone, phényle ou benzyle, et en ce qu'ensuite on sépare l'éther silylique.

**Claims for the Contracting States: BE CH LI DE FR GB IT LU NL SE**

1. Oestriol esters of the general formula I

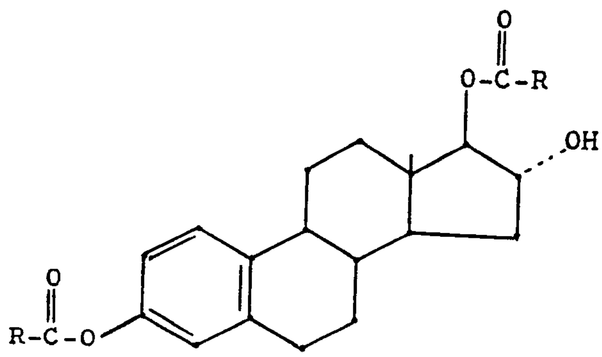

(I),

wherein R in each case represents the residue of a monocarboxylic acid having from 2 to 10 carbon atoms.

2. 3,17β-Diacetoxy-1,3,5(10)-oestratrien-16α-ol.
3. 3,17β-Dipropionyloxy-1,3,5(10)-oestratrien-16α-ol.
4. 3,17β-Diisobutyryloxy-1,3,5(10)-oestratrien-16α-ol.
5. 3,17β-Divaleryloxy-1,3,5(10)-oestratrien-16α-ol.
6. 3,17β-Dihexanoyloxy-1,3,5(10)-oestratrien-16α-ol.
7. 3,17β-Dipivaloyloxy-1,3,5(10)-oestratrien-16α-ol.
8. 3,17β-Didecanoyloxyoxy-1,3,5(10)-oestratrien-16α-ol.
9. 3,17β-Dibenzoyloxy-1,3,5(10)-oestratrien-16α-ol.
10. 3,17β-Dibutyryloxy-1,3,5(10)-oestratrien-16α-ol.
11. Pharmaceutical preparations, characterised in that they contain a compound according to claims 1 to 10.
12. Depot contraceptives, characterised in that they contain a compound according to claims 1 to 10.
13. A process for the preparation of novel oestriol esters of the general formula I

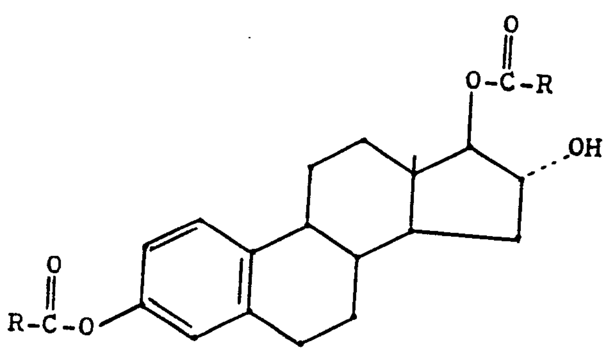

(I),

wherein R in each case represents the residue of a monocarboxylic acid having from 2 to 10 carbon atoms, characterised in that an oestriol-16-silyl ether of the general formula II

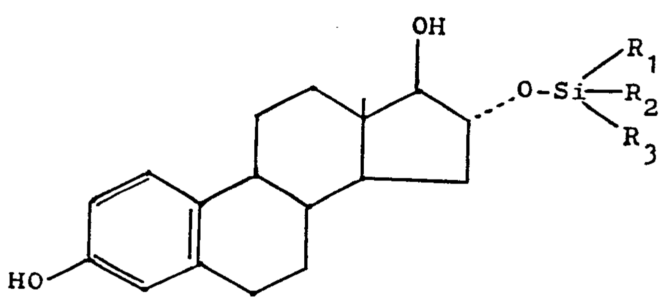

(II),

wherein $R_1$, $R_2$ and $R_3$ are the same or different, and each represents alkyl having from 1 to 5 carbon atoms, phenyl or benzyl, is esterified in a manner known *per se* in the 3 and 17 positions and the silyl ether is then cleaved.

**Claim for the Contracting State: AT**

A process for the preparation of novel oestriol esters of the general formula I

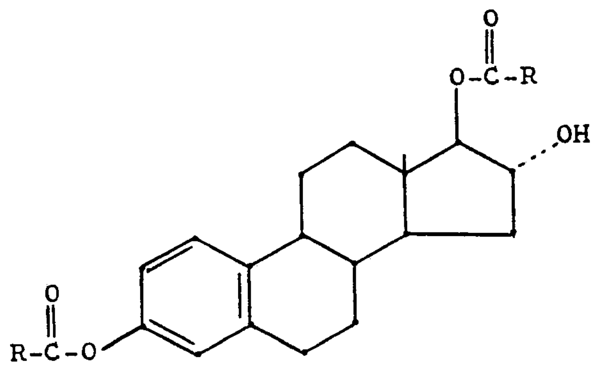

(I),

wherein R in each case represents the residue of a monocarboxylic acid having from 2 to 10 carbon atoms, characterised in that an oestriol-16-silyl ether of the general formula II

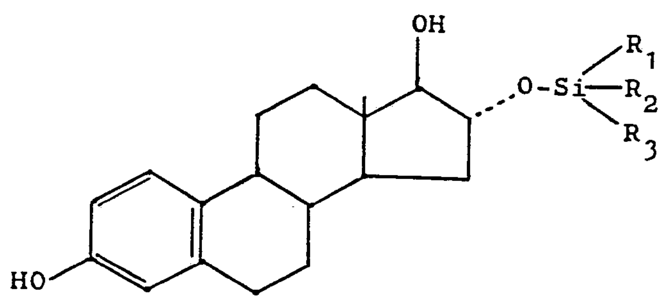

(II),

wherein $R_1$, $R_2$ and $R_3$ are the same or different, and each represents alkyl having from 1 to 5 carbon atoms, phenyl or benzyl, is esterified in a manner known *per se* in the 3 and 17 positions and the silyl ether is then cleaved.